# FASCICULE DE BREVET EUROPEEN

(11) **EP 2 549 942 B1**
(45) Date de publication et mention de la délivrance du brevet: **04.12.2013**
(21) Numéro de dépôt: 11712971.8
(22) Date de dépôt: 04.03.2011
(51) Int. Cl.: A61B 18/08, A61F 7/12, A61K 41/00, A61B 18/00, A61F 7/00

(54) **DISPOSITIF DESTINÉ À L'ADMINISTRATION DE CALORIES DANS UN TISSU, VAISSEAU OU CAVITÉ HUMAINE OU ANIMALE**
VORRICHTUNG ZUR FREISETZUNG VON KALORIEN IN GEWEBE, GEFÄSSEN ODER KAVITÄTEN BEI MENSCHEN ODER TIEREN
DEVICE FOR DELIVERING CALORIES INTO HUMAN OR ANIMAL TISSUE, VESSEL, OR CAVITY

(30) Priorité: 23.03.2010 FR 1052109
(43) Date de publication de la demande: 30.01.2013
(73) Titulaire: Nova Therma, 74400 Chamonix-Mont Blanc (FR)
(72) Inventeur: MEHIER, Henri, F-74400 Chamonix-Mont Blanc (FR)
(74) Mandataire: Denjean, Eric
(86) Numéro de dépôt international: PCT/FR2011/050462
(87) Numéro de publication internationale: WO 2011/117503

(56) Documents cités:
- DE-A1- 3 518 245
- FR-A1- 2 925 837
- US-A- 3 698 394
- US-A- 3 886 944
- US-A- 4 807 620
- US-A1- 2007 073 282

## Description

L'invention a pour objet un nouveau dispositif destiné à l'administration de calories, ou plus précisément de chaleur sèche, dans une collection liquidienne, dans une cavité (vessie etc..), un tissu ou vaisseau humain ou animal.

Le dispositif de l'invention peut être utilisé pour le traitement des tumeurs par technique dite de thermonécrose ou d'hyperthermie.

De manière connue, la thermonécrose entraine la destruction des cellules à une température supérieure à 65°C et s'adresse plus particulièrement aux tumeurs localisées. Cette technique est mise en oeuvre par chaleur sèche avec le laser, la RF, ou les ultrasons. Ces différents moyens sont utilisables en mode interventionnel aigue et la quantité importante d'énergie apportée en un temps court produit un phénomène de carbonisation qui peut provoquer des chocs mortels ce qui en fait conduit à n'utiliser ces techniques que sur des tumeurs de taille réduite. Avec la vapeur ce phénomène de carbonisation est minimisé, mais la diffusion possible de l'eau de condensation à haute température, limite l'application à des tumeurs situées dans des tissus homogènes et des organes de grande taille. Toutes ces méthodes n'arrivent pas à une nécrose complète et n'empêchent pas les récidives. Elles demeurent par ailleurs mal adaptées aux tumeurs localisées près des centres ou organes vitaux (cerveau, médiastin, sphère ORL..).

De son coté, l'hyperthermie est une technique consistant à augmenter la température des cellules à une valeur d'environ 45°C, ce qui n'entraine pas leur destruction mais augmente leur capacité de défense. Cette technique s'applique plus particulièrement aux tumeurs présentant des épanchements liquidiens. Elle est mise en oeuvre au moyen d'ondes courtes ou par liquides réchauffés par circulation extracorporelle

Il y a donc un intérêt à mettre au point un dispositif unique qui puisse être mis en oeuvre pour les deux technologies en programmant à la fois la température d'administration des calories , le temps et les périodes d'administration, comme cela est fait en chimio et radiothérapie, pour optimiser l'effet désiré et cela de façon ambulatoire, sans que le patient soit obligé de subir une nouvelle intervention et une nouvelle hospitalisation. Il y a donc un intérêt à mettre au point une méthode ambulatoire, qui permette de détruire progressivement et de façon programmée, des tumeurs même de taille importante et placées près de centres vitaux, en ayant le temps de contrôler l'effet de la thermonécrose ou de l'hyperthermie, par imagerie et en plaçant à la périphérie de la tumeur une sonde pour mesurer la température des tissus sains avoisinants.

Le dispositif de l'invention peut également être utilisé pour le traitement du vieillissement cutané en particulier pour le traitement des rides par régénération du derme en créant une inflammation par effet thermique qui entraine une cicatrisation avec régénération du collagène et des myofibrilles.

Le dispositif de l'invention peut enfin être utilisé pour le traitement des varices en créant un phénomène de fibrose ou de thermofibrose dans la paroi de la veine aboutissant à une oblitération de celle-ci.

Jusqu'alors, le Demandeur a mis au point un certain nombre de dispositifs décrits dans les documents WO 00/29055, WO 03/070302, WO 2006/108974 permettant d'injecter des calories par administration de vapeur d'eau dans des tissus ou des vaisseaux. Le principe général commun à l'ensemble de ces dispositifs est de maintenir l'eau avant sa libération, sous forme pressurisée, c'est-à-dire liquide à une température supérieure à 100°C, ce qui permet d'avoir un transfert de calories optimisé entre la source de chaleur et l'eau sous forme pressurisée. Pour ce faire, dans toutes les solutions proposées par le Demandeur jusqu'alors, l'eau circule dans un espace restreint, en pratique dans la lumière d'un microtube de diamètre compris entre 100 et 250 µm.

Plus récemment, le Demandeur a mis au point un dispositif différent et décrit dans le document WO2009/083688. Il s'agit d'une micro résistance qui vient chauffer l'eau présente dans la tumeur ou dans le sang contenu dans des veines variqueuses et donc sans apport d'eau externe. La microrésistance se présente sous la forme d'un fil de constantan central recouvert, à l'exception de son extrémité distale, d'une gaine de cuivre, puis sur toute sa longueur d'une couche isolante et d'un tube conducteur de courant. Un tel dispositif permet de générer de la chaleur exclusivement à l'extrémité distale de la micro résistance. Les inconvénients majeurs du système à part son coût plus élevé, sont que l'élément chauffant est central et la chaleur doit traverser l'élément isolant pour diffuser dans l'organisme avec une perte de rendement thermique et de fiabilité. Cet élément chauffant étant interne, l'utilisateur ne peut pas définir lui-même la longueur de chauffe ou la position de zones non chauffantes.

Dans tous les systèmes décrits par le Demandeur, l'énergie est fournie par un générateur standard non autonome agencé à l'extérieur de l'organisme, interdisant la mise en oeuvre du dispositif en ambulatoire.

Le document DE 35 18 245 montre un dispositif de chauffe implantable dans un vaisseau sanguin sous la forme d'un câble coaxial comprenant un fil central de faible résistance isolé par une gaine isolante. L'extrémité distale du fil est en contact avec un premier tressage conducteur de résistance élevé, lui-même en contact avec un deuxième tressage conducteur de faible résistance. Le deuxième tressage est dénudé de façon à exposer une partie du premier tressage servant d'élément de chauffe lorsque qu'un générateur est connecté entre le fil central et le deuxième tressage.

Le document US 3 698 394 montre un dispositif de chauffe implantable dans un vaisseau sanguin sous la forme d'une aiguille comprenant un fil de cuivre central à l'extrémité duquel est connecté un fil de résistance élevé, le tout inséré dans le tube de l'aiguille avec un isolant entre le fil et le tube de l'aiguille. L'extrémité distale du fil de résistance élevé est en contact avec le tube de l'aiguille, formant ainsi une élément de chauffe lorsqu'un générateur est connecté entre le fil central et le tube de l'aiguille.

L'objectif de l'invention est donc de simplifier ce type de système pour en faire un dispositif unique, de faible coût, qui soit adapté à tous les types de traitements décrits précédemment.

Un autre objectif de l'invention est de mettre au point un dispositif qui puisse être utilisé en ambulatoire, c'est-à-dire qui soit facilement transportable et donc autonome.

Enfin, aucun des appareils décrits précédemment n'est apte à une utilisation en multi thérapie. En d'autres termes, un troisième objectif de l'invention est de mettre au point un système unique qui soit non seulement capable de délivrer des calories sous la forme de chaleur sèche mais également de dispenser un traitement thérapeutique standard sous la forme de principe actif chimique ou radioactif.

Pour ce faire, le Demandeur à mis au point un nouveau système, toujours sous la forme d'une micro résistance, dont la longueur de la zone chauffante peut être déterminée en fonction et au moment de l'utilisation.

Plus précisément, l'invention a pour objet un dispositif en tout ou partie implantable destiné à l'administration de calories dans une cavité, un tissu ou un vaisseau notamment, comprenant:
- un fil de cuivre isolé, à l'exception de ses extrémités distale et proximale, sur au moins partie de la longueur duquel est serti un tube résistant présentant une résistivité supérieure à 20 µΩ.cm,
- le fil de cuivre est en contact par son extrémité distale avec ledit tube tandis que son extrémité proximale est destinée à être connectée à un générateur de courant,
- le tube est entouré sur partie de sa longueur d'au moins un cylindre mobile proximal apte à être serti sur le tube par l'utilisateur notamment, à l'aide par exemple d'une pince, ledit cylindre présentant une résistance électrique inférieure à 20 µΩ.cm,
- le cylindre mobile proximal et l'extrémité proximale du fil de cuivre présentent des moyens de connexion à un générateur de courant, en pratique l'extrémité proximale du fil de cuivre est reliée au pôle positif du générateur et le cylindre mobile est relié au pôle négatif du générateur par le biais par exemple d'un second fil de cuivre isolé.

En d'autres termes, l'invention consiste à sertir au moins en partie, sur un fil isolé amenant le courant, un matériau ramenant le courant et présentant une résistivité élevée se manifestant par un phénomène de chauffage. Sur ce matériau, est prévu un cylindre ou bague mobile de résistivité inférieure, non chauffant car réalisant un « shunt électrique » du circuit résistant, la position de la bague étant définie au moment de l'utilisation avant d'être sertie au moyen d'une pince par l'utilisateur, de manière définitive. Le cylindre sera positionné avantageusement au niveau de la peau et jusqu'à la zone à chauffer, après avoir délimité la taille de la tumeur ou de la veine et donc de la zone à chauffer.

Selon l'invention, le tube résistant présentant une résistivité supérieure à 20 µΩ.cm est serti sur le fil de cuivre par tréfilerie ou avantageusement par microemboutissage, lors de la fabrication. Dans un mode de réalisation préféré, le fil est serti par microemboutissage pour conduire à un profil triangulaire de l'ensemble fil/tube.

Le tube est serti au moins en partie sur le fil. Dans un mode de réalisation avantageux, le tube est serti sur le fil uniquement au niveau de son extrémité distale, de préférence sur une longueur d'environ 5 cm.

Dans un mode de réalisation perfectionné, le dispositif est muni d'un second cylindre mobile dénommé « cylindre distal » apte à être serti de la même façon, au moment de l'utilisation, le cylindre présentant lui-même à sa surface un support poreux diffuseur de principe actif.

Dans un autre mode de réalisation, le dispositif présente un second cylindre mobile dénommé « cylindre distal », dont la surface est munie d'un produit radioactif à visée thérapeutique et de localisation.

Dans les deux cas, le cylindre en tant que tel peut être construit en un matériau de résistivité variable, permettant de lui conférer un caractère plus ou moins chauffant en fonction de la nature de la zone à traiter et pour activer éventuellement la diffusion de l'actif.

Bien entendu, le dispositif de l'invention peut être muni de plusieurs cylindres permettant d'associer au traitement thermique, de la diffusion de principe actif chimique ou de la radiothérapie. Dans tous les cas, le cylindre le plus proximal est connecté au générateur de courant.

Dans cette hypothèse, l'opérateur dispose d'un système apte à la multithérapie, capable donc de délivrer à la fois de la chaleur sèche et un principe actif.

Selon l'invention, le tube présentant une résistivité supérieure à 20 µΩ.cm est réalisé dans un matériau choisi dans le groupe comprenant l'acier, l'inox, le nitinol ou le titane.

Le tube est comme déjà dit serti par tréfilerie ou par micro-emboutissage sur un fil de cuivre isolé dont l'extrémité distale non isolée est en contact avec l'extrémité distale dudit tube. Avantageusement, le fil de cuivre est isolé au moyen d'une couche de polyimide, teflon®, ou alumine.

Dans la suite de la description, le terme « distale » désigne l'extrémité du tube en contact avec la zone à traiter tandis que l'extrémité « proximale » désigne la partie du tube destinée à être connectée au générateur de courant.

Selon une autre caractéristique, le matériau constitutif du cylindre mobile présentant une résistivité électrique inférieure à 20 µΩ.cm est choisi dans le groupe comprenant le laiton ou le cuivre, éventuellement recouvert d'une très fine couche d'un matériau de meilleure biocompatibilité comme l'or déposé par électrochimie en « dorure flash ».

Bien entendu, le tube doit présenter un diamètre suffisamment faible pour pouvoir être incorporé dans l'organisme, en particulier dans les vaisseaux ou tissus. En pratique, le diamètre du dispositif est compris entre 0.3 et 0.7 mm, avantageusement de l'ordre de 0.5 mm, y compris la surépaisseur créée par la présence du cylindre mobile après sertissage.

Dans un mode de réalisation particulier, le tube se présente sous la forme d'une spirale. Cette forme est plus particulièrement adaptée pour traiter un volume plus important sans avoir recours à de multiples mises en place de microrésistances. Dans ce cas, le diamètre du dispositif est compris entre 1 et 1.2 mm, de manière à conférer une certaine rigidité à la spirale.

Selon une caractéristique essentielle, le tube est connecté à un générateur de courant. En fonction du type d'indication envisagée (varice, tumeur, ride), le chauffage du tube pourra être effectué en continu ou par pulse.

L'invention a donc également pour objet une installation associant un générateur de courant au dispositif tel qu'il vient d'être décrit.

De manière générale, le générateur se présente sous la forme d'un générateur électrochimique de type « pile alcaline » ou plus évoluée, qui présente l'avantage d'être autorégulée en courant et en tension, donc en puissance, éliminant tout danger pour l'organisme sans recours à un système électronique complexe. La pile est avantageusement une pile alcaline. Dans un mode de réalisation perfectionné, le fonctionnement de la pile est programmé.

Le générateur électrochimique présente également l'intérêt d'avoir un rapport poids/énergie, bien supérieur aux autres systèmes autonomes pour un coût de revient très faible par intervention. Suivant le type de traitement envisagé et donc le type de dispositif de l'invention (également désigné « microrésistance »), la tension de base est ajustée par le nombre de piles mises en service en série.

Pour générer des pulses de courant, le circuit électrique est complété par une « supercapacité » placée en parallèle entre le générateur de courant et la microrésistance. Le circuit contient dans ce cas un interrupteur ou un programmateur, fonctionnant en mode inverseur assurant la charge de la supercapacité puis sa décharge dans la microrésistance. Avec ce système, l'énergie délivrée par chaque pulse est parfaitement définie et toujours la même, sans recours à un système électronique complexe. La « supercapacité » généralement de type DLC (double layer electric field), présentant une capacité électrique très importante, de quelques Farads à plusieurs centaines de farads, bien adaptée aux besoins de ce dispositif médical et un nombre de charge et décharge pratiquement illimité.

Dans tous les cas, le générateur est avantageusement autonome.

L'invention peut être appliquée dans une méthode de traitement des rides par régénération du derme mettant en oeuvre le dispositif de l'invention.

Plus précisément, la méthode consiste :
- à définir la taille de la ride à traiter et donc la longueur de la partie chauffante du dispositif de l'invention à prévoir,
- à sertir à l'extrémité proximale de la zone chauffante ainsi déterminée, sur la partie restante du tube, le cylindre mobile proximal de résistivité inférieure à 20 µΩ.cm,
- à introduire sous la peau, au niveau de la ride, ou à positionner à la surface de ladite ride, la partie chauffante du dispositif,
- à connecter le dispositif aux bornes d'un circuit comprenant une pile, un générateur et un interrupteur de sorte à générer des pulses de courant,
- à déclencher ensuite le générateur de sorte à délivrer des pulses d'énergie compris entre 20 et 50 joules pendant une durée variant entre une et cinq secondes,
- à retirer le dispositif

Le générateur se présente en pratique sous la forme de 3 piles R20 délivrant une tension de 4.5V avec 18 AH permettant de délivrer environ 300 KJ, soit 6000 pulses. Le système de supercapacité est constitué de deux supercapacités de 22 farads disposées en série.

Dans la pratique, les pulses de courant créent un micro traumatisme engendrant une inflammation et un phénomène de cicatrisation ce qui permet de régénérer le collagène et les microfibrilles.

De la même façon que précédemment, le dispositif de l'invention peut être utilisé pour le traitement des tumeurs localisées, en cancérologie, par thermonécrose ou par hyperthermie.

Dans ce cas, on détermine la longueur du tube devant être chauffée en fonction de la taille de la tumeur. Le tube est alors muni d'un cylindre mobile proximal serti au moment de l'utilisation au passage de la peau. Dans un mode de réalisation perfectionné, le dispositif est en outre muni de cylindres mobiles distales additionnels présentant un support diffuseur de principe actif permettant d'assurer une multithérapie.

L'invention peut être appliquée dans un procédé de traitement des tumeurs par administration de calories consistant :
- à définir la taille de la tumeur à traiter et donc la longueur de la partie chauffante du dispositif de l'invention à prévoir,
- à sertir à l'extrémité proximale de la zone chauffante ainsi déterminée, sur la partie restante du tube, le cylindre mobile proximal de résistivité inférieure à 20 µΩ.cm,
- à inciser la peau,
- puis à introduire dans le tissu cible la partie chauffante du dispositif,
- puis, à déclencher ensuite le générateur de sorte à délivrer des pulses d'énergie,
- à retirer le dispositif en fin de traitement.

Dans certains cas, le dispositif est introduit dans un cathéter mis en place au préalable dans la tumeur permettant ainsi d'utiliser une « voie d'abord » déjà mise en place

De la même façon que précédemment, le dispositif de l'invention peut être utilisé pour le traitement des varices. Le principe est de chauffer directement le vaisseau en générant une quantité de calories suffisante pour permettre d'augmenter la température de l'eau contenue dans le vaisseau à une température d'environ 95°C.

Dans ce mode de traitement, le dispositif est introduit dans la veine au sein d'un cathéter résistant à la chaleur. La veine peut ainsi être vidée de son sang lequel peut être remplacé par de l'eau amenée par ce même cathéter permettant ainsi de créer une diffusion homogène d'énergie. Un contrôle permanent par échodoppler permet en temps réel de préciser le moment et l'état de l'oblitération de la veine. A la fin de l'opération, le dispositif est retiré en une seule fois.

Plus précisément, la méthode de traitement consiste :
- à effectuer un repérage de la veine à traiter, par marquage sur la peau du cheminement de la veine et de l'orifice d'introduction du dispositif,
- à définir la taille de la veine à traiter et donc la longueur de la partie chauffante du dispositif de l'invention à prévoir,
- à sertir à l'extrémité proximale de la zone chauffante ainsi déterminée, sur la partie restante du tube, le cylindre mobile proximal de résistivité inférieure à 20 µΩ.cm,
- à introduire dans la veine la partie chauffante du dispositif,
- à connecter le dispositif aux bornes du générateur,
- à déclencher ensuite le générateur de sorte à délivrer de l'énergie en continu compris entre 100 et 150 joules par centimètre de veine à traiter permettant de parvenir à l'oblitération de la veine.

L'invention et les avantages qui en découlent ressortiront bien des exemples de réalisation suivants à l'appui des figures annexées.

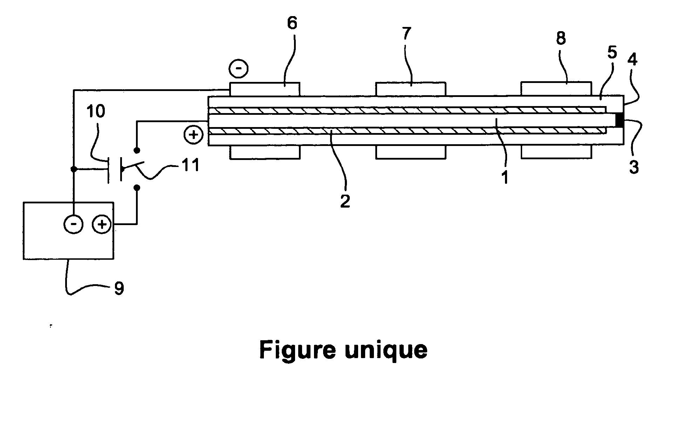

La figure 1 est une représentation schématique en coupe de l'installation de l'invention.

Le dispositif est constitué d'un fil de cuivre central (1) isolé par une couche de Polyimide (2). L'extrémité distale (3) du fil de cuivre est dénudée et en contact avec l'extrémité distale (4) du tube (5) réalisé en acier inoxydable. Le tube (5) est lui même revêtu de 3 bagues (6, 7, 8) mobiles. Chacune des 3 bagues est fabriquée en laiton. Les bagues sont serties au moment de l'utilisation après avoir défini la partie du tube à chauffer et donc l'emplacement restant pour les bagues. En pratique la bague (6) est positionnée au passage de la peau afin d'éviter tout risque de brûlure. La bague (7) est quant à elle munie d'un support poreux diffuseur de principe actif, non représenté. Enfin, la bague (8) dont la surface est munie d'un produit radioactif.

L'installation de l'invention contient en outre un générateur electrochimique de courant (9) ainsi qu'une supercapacité (10).

Le cheminement du courant est représenté au moyen de flèches sur la figure 1. L'extrémité proximale du fil de cuivre 1 est connectée au pôle plus d'un générateur par le biais du fil de cuivre alors que le pôle moins du générateur est relié à la bague proximale (5), la plus proche.

Le générateur se présente sous la forme de piles alcalines. Lorsqu'il est utilisé en mode pulsé, le générateur est associé à une supercapacité (10) en parallèle sur le circuit dont la charge et la décharge dans le dispositif sont gérés par le biais d'un interrupteur (11) ou d'un programmateur fonctionnant en mode inverseur

Comme déjà dit, le dispositif peut être mis en oeuvre pour des traitements divers que ce soit rides, varices ou encore cancérologie. Un autre avantage de l'invention est que le dispositif est doté d'une batterie autonome permettant non seulement de réduire le coût mais également de pouvoir assurer un traitement en ambulatoire.

## Revendications

1. Dispositif en tout ou partie implantable destiné à l'administration de calories dans une cavité, un tissu ou un vaisseau notamment, comprenant :
- un fil de cuivre isolé (1), à l'exception de ses extrémités distale et proximale, sur au moins partie de la longueur duquel est serti un tube (5) résistant présentant une résistivité supérieure à 20 µΩ.cm,
- le fil de cuivre (1) étant en contact par son extrémité distale (3) avec ledit tube tandis que son extrémité proximale est destinée à être connectée à un générateur de courant (9),
- le tube étant entouré sur partie de sa longueur d'au moins un cylindre mobile proximal (6) apte à être serti sur le tube (5), ledit cylindre (6) présentant une résistance électrique inférieure à 20 µΩ.cm,
- le cylindre mobile proximal (6) et l'extrémité proximale du fil de cuivre présentant des moyens de connexion à un générateur de courant (9).

2. Dispositif selon la revendication 1, **caractérisé en ce que** le tube (5) est serti sur le fil uniquement au niveau de l'extrémité distale dudit fil, de préférence sur une longueur d'environ 5 cm.

3. Dispositif selon la revendication 1, **caractérisé en ce que** le tube (5) est serti par micro emboutissage.

4. Dispositif selon la revendication 1, **caractérisé en ce qu'**il est muni d'un cylindre mobile distal (7, 8) muni sur sa surface d'un support poreux diffuseur de principe actif.

5. Dispositif selon la revendication 1, **caractérisé en ce qu'**il est muni d'un cylindre mobile distal (7, 8) dont la surface est munie d'un produit radioactif à visée thérapeutique et de localisation.

6. Dispositif selon la revendication 1, **caractérisé en ce que** le tube (5) présentant une résistivité supérieure à 20 µΩ.cm est réalisé dans un matériau choisi dans le groupe comprenant l'acier, l'inox, le nitinol ou le titane.

7. Dispositif selon la revendication 1, **caractérisé en ce que** le fil de cuivre (1) est isolé au moyen d'une couche (2) de polyimide, téflon®, ou alumine.

8. Dispositif selon la revendication 1, **caractérisé en ce que** le matériau constitutif du cylindre mobile proximal (6) présentant une résisistivité électrique inférieure à 20 µΩ.cm est choisi dans le groupe comprenant le laiton ou le cuivre.

9. Dispositif selon la revendication 1, **caractérisé en ce qu'**il présente un diamètre compris entre 0.3 et 0.7 mm, avantageusement de l'ordre de 0.5 mm.

10. Installation associant le dispositif objet de l'une des revendications 1 à 9 à un générateur de courant (9).

11. Installation selon la revendication 10, **caractérisée en ce que** le générateur de courant est un générateur electrochimique.

12. Installation selon la revendication 10, **caractérisé en ce qu'**elle comprend une supercapacité (10) placée en parallèle entre le générateur de courant (9) et le dispositif ainsi qu'un interrupteur (11) ou un programmateur, fonctionnant en mode inverseur assurant la charge de la supercapacité (10) puis sa décharge dans le dispositif.

## Patentansprüche

1. Ganz oder teilweise implantierfähige Vorrichtung zur Freisetzung von Kalorien insbesondere in Kavitäten, Gewebe oder Gefäße, umfassend:
- einen isolierten Kupferdraht (1), der mit Ausnahme seiner distalen und proximalen Enden, bei dem wenigstens auf einem Teil der Länge eine widerstandsfähige Röhre (5) eingespannt ist, die eine größere Resistivität als 20 µΩ.com aufweist,
- wobei der Kupferdraht (1) durch sein distales Ende (3) mit der genannten Röhre in Kontakt ist, während sein proximales Ende dazu bestimmt ist, an einen Stromgenerator (9) angeschlossen zu werden,
- wobei die Röhre wenigstens über einen Teil ihrer Länge mit einem proximalen mobilen Zylinder (6) umgeben ist, der geeignet ist, auf der Röhre (5) eingespannt zu werden, wobei der genannte Zylinder (6) einen elektrischen Widerstand von weniger als 20 µΩ.com aufweist,
- wobei der proximale mobile Zylinder (6) und das proximale Ende des Kupferdrahtes Anschlussmittel an einen Stromgenerator (9) aufweisen.

2. Vorrichtung gemäß Anspruch 1, **dadurch gekennzeichnet, dass** die Röhre (5) auf dem Draht nur an dem distalen Ende des genannten Drahtes, vorzugsweise über eine Länge von rund 5 cm, eingespannt ist.

3. Vorrichtung gemäß Anspruch 1, **dadurch gekennzeichnet, dass** die Röhre (5) per Mikro-Tiefziehen eingespannt ist.

4. Vorrichtung gemäß Anspruch 1, **dadurch gekennzeichnet, dass** sie mit einem distalen, mobilen Zylinder (7, 8) versehen ist, der auf seiner Oberfläche mit einem porösen Diffuserzylinder eines Wirkstoffs versehen ist.

5. Vorrichtung gemäß Anspruch 1, **dadurch gekennzeichnet; dass** sie mit einem distalen mobilen Zylinder (7, 8) versehen ist, dessen Oberfläche mit einem radioaktiven Produkt mit therapeutischer und lokalisierender Zweckbestimmung versehen ist.

6. Vorrichtung gemäß Anspruch 1, **dadurch gekennzeichnet, dass** die Röhre (5), die eine höhere Resistivität als 20 µΩ.com aufweist, aus einem Material realisiert ist, das aus der Gruppe ausgewählt ist, die Stahl, Edelstahl, Nitinol oder Titan umfasst.

7. Vorrichtung gemäß Anspruch 1, **dadurch gekennzeichnet, dass** der Kupferdraht (1) mittels einer Schicht (2) aus Polyimid, Teflon® oder Tonerde isoliert ist.

8. Vorrichtung gemäß Anspruch 1, **dadurch gekennzeichnet, dass** das den proximalen mobilen Zylinder (6) bildende Material, das eine elektrische Resistivität von weniger als 20 µΩ.com aufweist, aus der Gruppe ausgewählt ist, die Messing oder Kupfer umfasst.

9. Vorrichtung gemäß Anspruch 1, **dadurch gekennzeichnet, dass** sie einen Durchmesser aufweist, der zwischen 0,3 und 0,7 mm inbegriffen ist, vorteilhaft in der Größenordnung von 0,5 mm.

10. Anlage, die die Vorrichtung gemäß Anspruch 1 bis 9 einem Stromgenerator (9) zuordnet.

11. Anlage gemäß Anspruch 10, **dadurch gekennzeichnet, dass** der Stromgenerator ein elektrochemischer Generator ist.

12. Anlage gemäß Anspruch 10, **dadurch gekennzeichnet, dass** sie eine Superkapazität (10) umfasst, die parallel zwischen dem Stromgenerator (9) und der Vorrichtung platziert ist, sowie einen Schalter (11) oder einen Programmierer, der im Umkehrmodus funktioniert, der die Ladung der Superkapazität (10) und dann seine Entladung in die Vorrichtung gewährleistet.

## Claims

1. Wholly or partly implantable device intended for the administration of heat into a cavity, tissue or vessel in particular, comprising:
- a copper wire (1) that is insulated except at its distal and proximal extremities over at least part of the length of which there is crimped a resistant tube (5) having a resistivity of more than 20 µΩ.cm,
- the copper wire (1) being in contact with the said tube through its distal extremity (3) while its proximal extremity is intended to be connected to a current generator (9),
- the tube being enclosed over part of its length by at least one proximal movable cylinder (6) which can be crimped onto the tube (5) the said cylinder (6) having an electrical resistance of less than 20 µΩ.cm,
- the movable proximal cylinder (6) and the proximal extremity of the copper wire having means for connection to a current generator (9).

2. Device according to claim 1, **characterised in that** the tube (5) is only crimped onto the wire at the distal extremity of the said wire, preferably over a length of approximately 5 cm.

3. Device according to claim 1, **characterised in that** the tube (5) is crimped by microswaging.

4. Device according to claim 1, **characterised in that** it is provided with a distal movable cylinder (7, 8) provided on its surface with a porous support which is a diffuser for an active ingredient.

5. Device according to claim 1, **characterised in that** it is provided with a distal movable cylinder (7, 8) provided on its surface with a radioactive product for therapeutic and location purposes.

6. Device according to claim 1, **characterised in that** the tube (5) which has a resistivity of more 20 µΩ.cm is made of a material selected from the group comprising steel, stainless steel, nitinol or titanium.

7. Device according to claim 1, **characterised in that** the copper wire (1) is insulated by means of a layer (2) of polyimide, teflon®, or alumina.

8. Device according to claim 1, **characterised in that** the material comprising the proximal movable cylinder (6) having an electrical resistivity of less than 20 µΩ.cm is selected from the group comprising brass or copper.

9. Device according to claim 1, **characterised in that** it has a diameter of between 0.3 and 0.7 mm, advantageously of the order of 0.5 mm.

10. Equipment combining the device according to any one of claims 1 to 9 with a current generator (9).

11. Equipment according to claim 10, **characterised in that** the current generator is an electrochemical generator.

12. Equipment according to claim 10, **characterised in that** it comprises a supercapacitance (10) placed in parallel between the current generator (9) and the device together with a switch (11) or programmer operating in inverter mode ensuring that the supercapacitance (10) charges up and then discharges in the device.
